(19) Europäisches Patentamt
European Patent Office
Office européen des brevets

(11) **EP 2 267 167 A1**

(12) **EUROPEAN PATENT APPLICATION**
published in accordance with Art. 153(4) EPC

(43) Date of publication:
**29.12.2010 Bulletin 2010/52**

(51) Int Cl.:
*C12Q 1/70* (2006.01)    *G01N 33/00* (2006.01)

(21) Application number: **09723530.3**

(22) Date of filing: **17.03.2009**

(86) International application number:
**PCT/ES2009/070066**

(87) International publication number:
**WO 2009/115633 (24.09.2009 Gazette 2009/39)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **17.03.2008  ES 200800776**

(71) Applicants:
• **Consejo Superior De Investigaciones Científicas (75%)**
  **28006 Madrid (ES)**
• **Universidad De Barcelona (25%)**
  **08007 Barcelona (ES)**

(72) Inventors:
• **GARCÍA ALJARO, Cristina**
  **E-08193 Bellaterra (Barcelona) (ES)**
• **MUÑOZ BERBEL, Xavier**
  **E-08193 Bellaterra (Barcelona) (ES)**
• **MUÑOZ PASCUAL, Francisco Javier**
  **E-08193 Bellaterra (Barcelona) (ES)**
• **BLANCH GISBERT, Anicet R.**
  **E-08007 Barcelona (ES)**

(74) Representative: **Pons Ariño, Angel**
  **Glorieta Ruben Dario 4**
  **28010 Madrid (ES)**

(54) **METHOD AND SYSTEM FOR DETECTING AND/OR QUANTIFYING BACTERIOPHAGES THAT CAN INFECT A PREDETERMINED BACTERIAL HOST STRAIN, USE OF A MICROELECTRONIC SENSOR DEVICE FOR DETECTING SAID BACTERIOPHAGES AND MICROELECTRONIC SENSOR DEVICE FOR IMPLEMENTING SAID METHOD**

(57)    The method, system and device claimed are based on the measurement of changes in impedance arising on the interface of an electrode to which bacteria of a host strain for the bacteriophage to be detected have previously been made to adhere. The changes arising in said electrode/bacteria interface originate from the phagic activity of the bacteriophages on the bacteria adhering to the surface of the working electrode of a microelectronic sensor device.

FIG.1

**Description**

The present invention relates to a process and system for detecting and/or quantifying bacteriophages capable of infecting a predetermined bacterial host strain, which is based on measuring the electrical impedance of the medium or solution being analysed using the electrical impedance spectroscopy technique.

It also relates to a microelectronic sensor device for carrying out said process and the use of said microelectronic sensor device for detecting and quantifying said bacteriophages.

## BACKGROUND OF THE INVENTION

Bacteriophages are viruses that infect bacteria and are formed from nucleic acids (DNA or RNA) wrapped in a protein coat or "capsid". These viruses have high specificity for the bacteria they infect, in such a manner that a certain bacteriophage or group of bacteriophages can be detected based on the selected host strain.

In short, the bacteriophage cycle consists of four phases: in a first phase, bacteriophage-bacteria recognition and injection of phagic nucleic acids containing the necessary genetic information for forming new bacteriophages takes place. Next, the genetic material of the bacteriophages is integrated in the bacterial chromosome in the form of DNA. Once integrated, the infection of the host cell can provoke two types of response according to the type of bacteriophage it infects. We can therefore distinguish between temperate bacteriophages, which can become integrated in the genome of the host cell indefinitely or until lysis is induced by means of diverse factors, and lytic bacteriophages which, after an incubation period and thanks to the biosynthetic machinery of the host cell, replicate the necessary material for creating new virions and are released to the exterior via lysis of the host cell.

The detection and/or quantification of bacteriophages of enteric bacteria is of particular importance to the microbiological control of water and to determining the origin of fecal contamination. Said bacteriophages have been proposed as indicator microorganisms of the presence of viruses, as their behaviour is similar to these. There are three groups of bacteriophages of enteric bacteria which are use as indicators of fecal contamination of viral origin: somatic coliphages, F-specific RNA phages and phages infecting *Bacteroides spp.*

Another group of bacteriophages the detection and/or quantification of which is of particular interest, is that formed by bacteriophages infecting lactic bacteria of the type used to produce cheese or other lactic-type fermented products. Said bacteriophages seriously affect the production of lactic acid, entailing substantial economic losses in lactic industries.

The classical methods for detecting the presence of bacteriophages are based on the detection of their effects on their host bacteria. Traditionally, bacteriophages were quantified using a double-layer agar culture, wherein a semi-solid agar layer containing a certain host strain (according to the bateriophage being quantified) and the sample being analysed is deposited on an agar layer. Quantification of the bacteriophages is carried out through the detection of lysis areas or plaques originated by the infection and lysis of the host bacteria by a bacteriophage or group of bacteriophages present in the sample, after an incubation period of said host strain.

Methods based on the sowing of agar plates have the drawback of being very slow, as they require at least 24 hours of incubation.

Other, more recently described methods for detecting bacteriophages are based on the detection of said bacteriophages using the PCR technique. However, these methods are also slow and very painstaking.

European patents EP0149383 and EP0714450 describe other methods for detecting bacteriophages, specifically for detecting bacteriophages of lactic bacteria.

Patent EP0714450 relates to the detection of an internal component of a bacterium upon lysis using bioluminescence emission, in enzymatic reactions catalyzed by enzymes such as luciferase, while Patent IP0149383 relates to the detection of the inhibition of growth using pH indicators to detect microbial activity.

The methods described in the aforementioned patents have the drawback of not allowing the detection of bacteriophages in an active state, due to which they are not very reliable in practice. On the other hand, they are highly complex and expensive methods, as they entail the use of a large number of reactives.

Spanish patent application number 200800109, not published at the time of filing the application, relates to a process and device for measuring the concentration of biomass which uses the electrochemical impedance spectroscopy (EIS) technique to determine the change in impedimetric signal produced by the adhesion of the biomass onto the surface of the work electrode of a device.

In an embodiment described in the aforementioned patent application, the electrodes with which interface impedance measurements are performed are disposed integrated in a material substrate of a flat microelectronic device, specifically a chip sensor, susceptible to being immersed in the medium in which the biomass concentration in the solution is measured.

In the process and device disclosed in the aforementioned patent application, biomass concentration is determined based on the change in capacitance value of the electric double layer of the electrode-solution interface produced by the electrostatic adhesion of the biomass.

Capacitance variation of the electric double layer of the electrode-solution interface has been observed to depend on the biomass concentration in the solution, due to which monitoring the changes in the capacitance of said layer allows the detection of biomass concentration in the solution, in a simple and very reliable manner, by means of the corresponding calibration curve.

## SUMMARISED DESCRIPTION OF THE INVENTION

A first objective of the present invention is to develop an alternative process and system to detect and/or quantify bacteriophages based on measuring the changes in electrical impedance produced in the interface of an electrode whereonto bacteria have been previously adhered.

The described process and system have the advantage over the processes of the state of the art that it is very simple, inexpensive and highly reliable.

In accordance with this objective, according to a first aspect, the present invention provides a process for detecting and/or quantifying bacteriophages capable of infecting a predetermined bacterial host strain, **characterised in that** it comprises the stages of:

a) adhering bacteria from at least one host strain onto the surface of a work electrode of a device comprising means for measuring electrical impedance;

b) exposing the electrode and adhered bacteria to a solution of the material being analysed which is susceptible to containing bacteriophages;

c) incubating, together with the electrode, the solution of stage b) under predetermined conditions so that, if said bacteria are infected by bacteriophages, lysis of said bacteria adhered onto the electrode takes place;

d) measuring the change in electrical impedance of a solution, while carrying out the incubation of stage c),

e) e-i) determining, in the equivalent electrical circuit, the change in capacitance value of the electrode-bacteria interface, based on said change in impedance value; or

e-ii) determining the value of the change in magnitude of the imaginary impedance component at a predetermined frequency according to said host strain, based on the said change in impedance value; and

f) determining the presence or concentration of bacteriophages in the solution, as of said change in capacitance value or change in magnitude of the imaginary impedance component, by means of the corresponding calibration curve that correlates said change in capacitance value or change in magnitude value of the imaginary impedance component, with the bacteriophage concentration of the solution.

In accordance with the same objective, according to a second aspect, the present invention provides a system for detecting and/or quantifying bacteriophages capable of infecting a predetermined bacterial host strain, **characterised in that** it comprises a microelectronic sensor device comprising at least two electrodes for performing electrical impedance measurements, bacteria from at least one host strain adhered onto the surface of at least one of said electrodes, and processing and control means to determine either the change in capacitance of the electrode-bacteria interface, in the equivalent circuit, or the change in magnitude of the imaginary impedance component at a predetermined frequency according to said host strain, said change in capacitance or change in magnitude of the imaginary impedance component occurring as a result of the lysis of the bacteria adhered onto the electrode, on being said bacteria infected by bacteriophages.

A second objective of the present invention is that of providing a microelectronic sensor device comprising at least one pair of electrodes for measuring electrical impedance changes. Said sensor device is **characterised in that** it comprises a bacterial biofilm adhered onto the surface of at least one of said electrodes, the bacteria of said biofilm stemming from at least one host strain susceptible to being infected by bacteriophages of a predetermined specificity for said strain.

Finally, a third objective of the present invention is the use of a microelectronic sensor device, comprising at least one pair of electrodes integrated in a material substrate for detecting and quantifying, by means of electrochemical impedance spectroscopy, bacteriophages capable of infecting a predetermined bacterial host strain.

In the present invention, "microelectronic device" shall be understood to be a flat sensor component, preferably manufactured using thick-film or thin-film microelectronic technologies.

"Biofilm" shall be understood to be a heterogeneous bacterial formation growing on the surface of the work electrode of a microelectronic device; preferably a bacterial community growing embedded in an exopolysaccharide matrix adhered onto the surface of the work electrode of a microelectronic device.

## DETAILED DESCRIPTION OF THE INVENTION

The process and system being claimed are based on the measurement of the impedance changes produced in the interface of an electrode, whereonto bacteria of a host strain have previously been adhered for detecting the desired bacteriophage or group of bacteriophages. The changes produced in said electrode-bacteria interface are originated by the phagic action of the bacteriophages on the bacteria adhered onto the surface of the work electrode. Said electrode belongs to a microelectronic sensor device that measures impedances using electrochemical impedance spectroscopy.

The experiments conducted have revealed that the lysis carried out by bacteriophages of bacteria adhered onto the work electrode of a microelectronic sensor device produces a response that can be monitored in real time using electrical impedance means.

Specifically, it has been observed that the capacitance value of the interface of an electrode whereonto bacteria have adhered may be modified by the phagic activity of bacteriophages that provoke the lysis of said bacteria. The interface capacitance change value, determined in the equivalent electrical circuit of the impedance spectrum, depends on the degree of phagic activity (lysis) that takes place on the electrode surface, which in turn is proportional to the concentration of bacteriophages in the medium or solution being analysed.

Nevertheless, it has also been observed that the infection produced by the bacteriophages can be monitored in a faster and simpler manner by directly measuring the change in magnitude of the imaginary impedance component at a predetermined frequency. The imaginary impedance component is related to the capacitative component of the impedance of the circuit, which has also been observed to vary (descend) with the presence of bateriophages in the solution.

These facts have enabled the development of a system and process for detecting and/or quantifying bacteriophages having the advantage of being highly reliable, as it detects the bacteriophages in an active state. Additionally, it is a very simple method and system, easily reproduced and automated, which allows monitoring in real time of the bacteriophage concentration in a sample. On the other hand, it has the advantage of being applicable for detecting the bateriophages present in any type of matrix (sewage, sewage plant mud, leachate, biosolids, etc.).

Both the claimed system and process require the adhesion, onto the surface of the work electrode, of the host strain to detect the desired bateriophage or group of bacteriophages. Said bacterial adhesion may be carried out using different techniques.

According to a first embodiment, the adhesion can be carried out through the prior functionalisation of the work electrode surface with the adsorption of a compound or solution destined for immobilising the bacteria.

According to another embodiment, the adhesion can be carried out applying electric potential to the electrodes of the device, for the purpose of provoking the electrostatic adhesion of the bacteria onto the work electrode.

Nevertheless, according to a preferred embodiment, the bacterial adhesion comprises the formation of a bacterial biofilm on the surface of the work electrode which will, preferably, have a thickness of at least 25 microns or a concentration of $10^4$ PFU/ml (Plaque-Forming Units). It has been verified that said thickness or concentration of bacteria affords the methodology greater sensitivity.

The formation of a biofilm follows a characteristic process that starts with a reversible phase of bacterial adhesion onto a substrate, followed by an irreversible adhesion and the start of the bacterial division that will entail the growth and subsequent maturity of the biofilm. This process can be monitored using the electrochemical impedance spectroscopy technique, following an equivalent circuit wherein the capacitance of the biofilm (Cb) is defined as the parameter or electric component of said circuit that models the biofilm adhered onto the electrode in the interface. The capacitance of the biofilm shows a positive correlation with the thickness of the biofilm, in such a manner that it increases on increasing the thickness of the biofilm during maturity thereof.

Advantageously, the formation of the biofilm of the present invention comprises the exposure of the work electrode to a culture medium containing bacteria from the host strain, and the simultaneous polarisation of the work electrode for a predetermined period of time according to said host strain.

It has been observed that a compact biofilm can be formed in a matter of hours by applying said simultaneous polarisation during electrode incubation, which has a very positive effect on the process.

According to the preferred embodiment that includes the formation of the biofilm, the present invention claims a process wherein:

- in stage e-i), determining the change in capacitance of the interface comprises determining the capacitance (Cb) of said biofilm, said capacitance being the parameter of the equivalent electric circuit modelled by said biofilm in the interface,

and wherein, subsequent to stage e-i), the presence or concentration of bacteriophages is determined based on the change in capacitance (Cb) of said biofilm, said change in capacitance being correlated with the deterioration of said biofilm as a result of the lysis.

According to the same preferred embodiment that includes the formation of the biofilm, the present invention claims a system wherein the bacteria adhered to the work electrode of the microelectronic device form part of said biofilm and wherein the processing and control means determine either the change in capacitance (Cb) of said biofilm or the change in magnitude of the imaginary impedance component, said change in capacitance or change in magnitude of the imaginary impedance component being correlated with the deterioration of the same biofilm as a result of the bacterial lysis.

The experiments conducted have revealed that, when a biofilm formed on the work electrode of a microelectronic sensor device is exposed to a sample containing a bacteriophage solution, the bacterial lysis provoked by these produces a decrease in the thickness of said biofilm which results in either a change in capacitance (Cb) of said biofilm or a change in magnitude of the imaginary impedance component related to the capacitance of the circuit.

Surprisingly, it has been observed that both the change in thickness and the change in capacitance (Cb) of the biofilm or change in magnitude of the imaginary impedance component, can be associated with the lytic cycle of the bacteriophages responsible for removing the bacteria from the biofilm adhered onto the surface of the microelectronic device.

Based on the foregoing, the present invention provides a very simple and reliable system and process wherein, according to a preferred embodiment, the presence of bacteriophages in a solution is detected and quantified by detecting changes in impedance associated with the deterioration of a biofilm adhered onto the surface of a microelectronic device.

Also based on the foregoing, the present invention provides a microelectronic sensor device comprising at least one pair of electrodes for measuring changes in electric impedance, **characterised in that** it comprises a bacterial biofilm adhered onto the surface of at least one of said electrodes, the bacteria of said biofilm belonging to a host strain susceptible to being infected by bacteriophages of predetermined specificity for said strain.

Advantageously, the microelectronic device that includes a biofilm is used to detect and quantify, by means of electrochemical impedance spectroscopy, bacteriophages capable of infecting at least one host strain of the bacteria of said biofilm.

Preferably, the biofilm of the device has a thickness of at least 25 microns or a concentration of bacteria of said host strain of at least $10^4$ PFU/ml (Plaque-Forming Units).

Also preferably, the microelectronic device is a flat, miniaturised microelectronic sensor chip or device, preferably manufactured using thin-film lithographic technologies.

The chip constitutes a highly sensitive sensor which, due to its small size, enables measurements in very small sample volumes.

The process and system being claimed can be applied to the detection of any type of bacteriophages, provided that the host strain for which said bacteriophage has a predetermined specificity is available. Said bacteriophages may stem from samples of very diverse materials; for example, sewage plant mud, leachate or other types of solid or liquid materials wherein the detection of bacteriophages is of interest.

In the specific case of water samples, the detection of bacteriophages is of particular interest, as these have been proposed as indicator microorganisms of the presence of viral-type fecal contamination.

According to a preferred embodiment, the present invention relates to the detection of bacteriophages of importance to the microbiological control of water and/or the determination of fecal contamination, which include, for example, somatic coliphages, F-RNA specific bateriophages or bacteriophages infecting *Bacteroides fragilis*.

According to other embodiments, the bateriophages of the present invention are bacteriophages infecting *Pseudomonas putida* or bacteriophages infecting lactic bacteria, such as for example: *Lactobacillus bulgaris, Lactobacillus lactis, Lactobacillus helveticus, Lactobacillus plantarum* and *Streptococcus thermophilus*.

## BRIEF DESCRIPTION OF THE DRAWINGS

In order to better understand the foregoing, drawings have been attached wherein:

Figure 1 shows a schematic plan view of the structure of a microelectronic sensor device, specifically an impedimetrical sensor chip, used to detect bacteriophages.

Figures 2a and 2b show details of the chip of figure 1, whereon a biofilm of *Pseudomonas putida* has been made to grow. Figure 2a shows the surface of the work electrode of the chip with the biofilm before being subjected to the action of the bacteriophages, while figure 2b shows the same surface of the electrode after being subjected to the action of the bacteriophages infecting *Pseudomonas putida.*

Figure 3 is a graphic representation showing the evolution of the capacitance (Cb) of the biofilm grown on the chip of figure 1, in a control solution and in a solution comprising bacteriophages infecting *Pseudomonas putida.*

Figure 4 shows a representation of the equivalent electric circuit used to model biofilm capacitance (Cb) in the interface.

## DESCRIPTION OF PREFERRED EMBODIMENTS

A first embodiment of the process and system of the present invention for the detection of bacteriophages infecting *Pseudomonas putida* is described below.

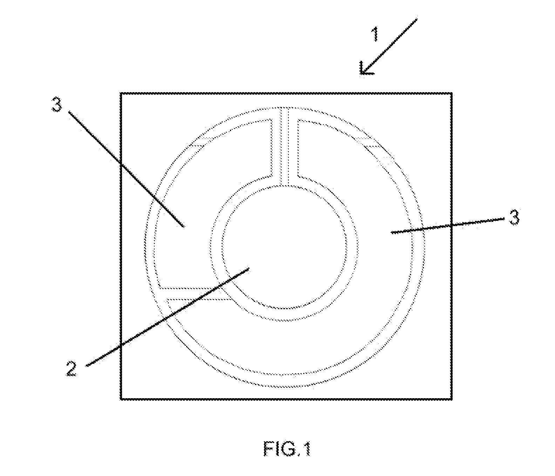

The microelectronic device used is a sensor chip 1 such as that represented in figure 1 integrating, in a single substrate, a work electrode 2 and two counter electrodes 3, all in the form of platinum micro-disks.

As mentioned in the detailed description of the invention, the process and system being claimed are based on the measurement of the changes in impedance produced in the interface of an electrode, whereonto bacteria of a host strain have previously been adhered in order to detect the desired bacteriophage or group of bacteriophages.

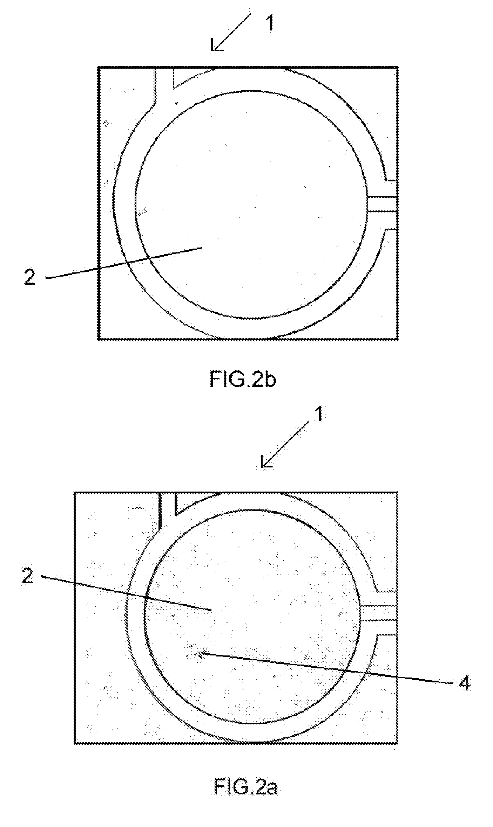

In the embodiment being described, the bacteria have been adhered onto the surface of the chip 1 of figure 1 through the formation of a biofilm 4 of *Pseudomonas putida.* Figure 2a shows a detail of the sensor chip of figure 1 whereon said biofilm 4 has been made to grow.

For the formation of the aforementioned biofilm 4, the sensor chip 1 itself was introduced in a reactor with a *Pseudomonas putida* culture in an exponential phase, in a controlled minimal medium (AB minimal medium) with agitation and constant ventilation. In this reactor, chip 1 was allowed to incubate for a period of five years. After this period, the biofilm 4 formed on the chip 1 has the appearance shown in figure 2a.

The composition and method for preparing the AB minimal medium prepared from a mixture of the following solutions are detailed below:

    ○ 875 ml of sterile distilled water
    ○ 100 ml of A solution
    ○ 25 ml of B solution
    ○ 10 ml of 20% glucose (20g/100ml of distilled water)

The A solution includes, in 1,000 ml of distilled water:

    ○ $(NH_4) SO_4$:20g
    ○ $Na_2HPO_4$:73.1 g
    ○ $KH_2PO_4$:78.5 g
    ○ NaCl:30.0 g
    ○ $NaSO_4$:0.11 g

The B solution includes the following components, which are mixed together after being individually autoclaved:

    ○ 16 g $MgCl_2$. 6 $H_2O$, in 500 ml of distilled water
    ○ 0.58g $CaCl_2$, in 250 mg of distilled water
    ○ 0.032 g $FeCl_3$, 6 $H_2O$, in 250 ml of distilled water

Once the biofilm 4 has been formed on the chip 1, said chip 1 is introduced into a solution containing a suspension of bacteriophages at $10^8$ (PFU/ml) (Plaque-Forming Units). This solution has been obtained on inoculating 0.5 ml of a bacteriophage-containing sewage sample, in 4.5 ml of said minimal AB medium.

Next, the aforementioned solution that includes the chip 1 is incubated for a period of 24 hours at a temperature of 37˚-C. During the incubation process, electric impedance measurements have been performed for the purpose of monitoring biofilm 4 deterioration.

The impedance measurements were performed using electrochemical impedance spectroscopy, applying the following measurement conditions:

    ■ Temperature: 37▯C
    ■ Culture medium: AB minimal medium, according to the aforementioned composition
    ■ Open circuit DC potential: +0.26 $\pm$ 0.5 V
    ■ AC potential: 25 mV
    ■ Frequency range: 10 Hz to 100 KHz

In this electrical environment, the biofilm 4 adhered onto the surface of the chip 1 behaves like a dielectric material which is modelled, in the equivalent electric circuit of the impedance spectrum, as biofilm capacitance (Cb). The equation defined by biofilm 4 capacitance (Cb) is given by the following formula:

$$C_{biofilm} = \varepsilon_0 \, \varepsilon_{biofilm} \, \frac{A}{d}$$

where $\varepsilon_0$ is the coefficient of permittivity in a vacuum, $\varepsilon_{biofilm}$ is the permittivity of the biofilm; $A$ is the area of the biofilm coating and $d$ is the thickness of the biofilm.

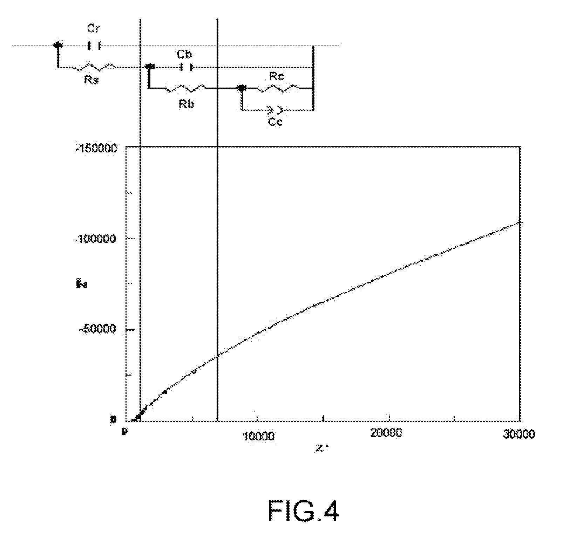

Figure 4 shows a representation of the aforementioned equivalent circuit wherein, in addition to biofilm capacitance (Cb), the following parameters are defined:

- ■ *Cr*. Capacitance associated with the aforementioned electrode.
- ■ *Rs*: Solution resistance.
- ■ *Rb*: Biofilm 4 resistance.
- ■ *Cc*: Capacitance of the electrical double layer of the interface.
- ■ *Rc*: Load transfer resistance.

As mentioned in the description of the invention, the exposure of the biofilm 4 to the action of the bacteriophages generates a response that is impedimetrically measurable in terms of variation in the equivalent electrical circuit parameter, i.e. in terms of variation in the capacitance of said biofilm 4.

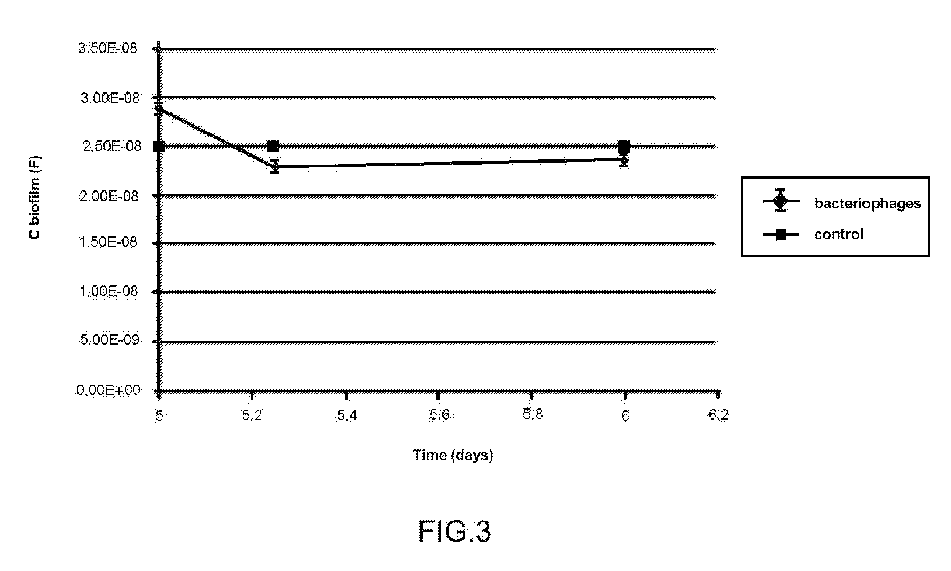

Figure 3 is a graphic representation showing the evolution over time of the capacitance (Cb) of the biofilm 4 disposed on the sensor chip 1, in a control solution and in a solution of a sewage water sample containing bacteriophages infecting *Pseudomonas putida.*

In said figure 3 it can be observed that biofilm 4 capacitance (Cb) decreases (8% compared to the control solution) six hours after incubating the chip 1 when disposed in a solution containing bacteriophages. However, the same capacitance (Cb) is maintained constant when the same chip 1 is disposed in a control solution without bacteriophages.

As mentioned in the description of the invention, the experiments conducted have demonstrated that the changes in biofilm 4 capacitance (Cb) are correlated with the changes in the thickness of the same biofilm 4, as could be observed using confocal laser microscopy. The change in these two parameters is associated with the lytic cycle of the bacteriophages responsible for removing the bacteria from the biofilm 4.

In the embodiment being described, the thickness of the biofilm 4 in the chip 1 which had been exposed to the action of the bacteriophages was measured, detecting a reduction of around 50% of the thickness of the biofilm, although the total reduction of the bacteria (UFC/ml) (Colony-Forming Units) was around 20%, probably due to the fact that only the outer layers of the biofilm 4, which have less bacterial density, were capable of being infected by the phages.

These measures for reducing the thickness of the biofilm 4 were correlated with the decrease in biofilm capacitance (Cb) produced by the presence of bacteriophages, as can be observed in figure 3.

The concentration of bacteriophages in the solution being analysed is determined based on the change in the biofilm capacitance (Cb) value by means of the corresponding calibration curve that correlates said capacitance (Cb) value change with the concentration of the solution within the work range.

Next, a second embodiment of the process and system of the present invention for detecting bacteriophages infecting *Escherichia coli is* described.

In this second embodiment, the microelectronic device used is the same sensor chip 1 of figure 1, whereon in this case a biofilm having a thickness of 30 microns and a bacterial concentration of $10^5$ PFU/ml has been formed.

For the rapid formation of a compact biofilm of the aforementioned thickness and bacterial concentration, the sensor chip 1 itself is introduced into a bioreactor with an *Escherichia Coli* culture in an exponential phase in a controlled medium (AB minimal medium) with agitation and constant ventilation. After three minutes of conditioning the sensor in the bioreactor, the chip 1 is subjected to forced incubation by polarising the work microelectrode using a voltage of three volts applied using a pulse train lasting ten seconds. Under these conditions, the biofilm with the aforementioned characteristics is obtained. This biofilm has the same appearance as the normal biofilm incubated spontaneously for several days shown in figure 2a.

Once the biofilm 4 is formed on the chip 1, the same infection protocol is followed as that described in the first embodiment, introducing the chip with the biofilm in a solution containing a suspension of bacteriophages of $10^8$ (PFU/ml) (Plaque-Forming Units). Subsequently, the aforementioned solution that includes the chip is incubated for a period of six hours at a temperature of 37 C. During the incubation process, electrochemical impedance measurements are performed for the purpose of monitoring biofilm deterioration.

As in the case of the first embodiment, the exposure of the biofilm to the action of the bacteriophages generates a response that is impedimetrically measureable in terms of variation in the equivalent circuit parameter modelled by the biofilm in the interface, i.e. in terms of variation in the capacitance (Cb) of said biofilm.

Table 1 shows the results obtained in this second embodiment for two types of bacteriophages capable of infecting the biofilm of *Escherichia coli* created on the microchip. The results are expressed as a percentage of reduction in the capacitance of said biofilm.

Table 1. - Percentage of reduction in the capacitance of the biofilm due to the action of the bacteriophages after two hours and six hours of incubation.

| BACTERIOPHAGE | % REDUCTION IN $C_{biofilm}$ SIGNAL | |
|---|---|---|
| | 2 h | 6 h |
| A*Escherichia coli* | 2 % | 24 % |
| B*Escherichia coli* | 3.5 % | 19 % |

As can be observed in the table above, for a bacteriophage A of *Escherichia coli,* after six hours of incubation the capacity signal of the biofilm was reduced 24%, while it was reduced 19% in the case of bacteriophage B.

In this second embodiment, the results show that the formation of a compact biofilm having a thickness of 30 microns and $10^4$ UFC/ml affords the methodology greater sensitivity. Said compact biofilm can be quickly formed (in a matter of hours) by subjecting the chip to forced incubation by means of simultaneous polarisation of the work electrode.

As already commented in the description of the invention, the exposure of the biofilm to the action of the bacteriophages generates a response that is also impedimetrically measurable in terms of variation in the magnitude of the imaginary impedance component (Zi) obtained at a certain frequency.

The results of a third embodiment of the process and system of the present invention for detecting bacteriophages infecting *Escherichia coli,* wherein the detection of bacteriophages is carried out based on the change in value of the magnitude of the imaginary impedance component, is described below.

In this third embodiment, the microelectronic device used is the same sensor chip 1 of figure 1, whereon a biofilm having a thickness of 40 microns and a bacterial concentration of $3.10^5$ PFU/ml has been formed.

Table 2 shows the results obtained in this third embodiment for two types of bacteriophages capable of infecting the biofilm of *Escherichia coli* created on the microchip. The results are expressed as a percentage of variation in the magnitude of the imaginary impedance component.

Table 2. - Percentage of variation in the magnitude of the imaginary impedance component due to the action of the bacteriophages after six hours of incubation.

| BACTERIOPHAGE | % VARIATION $Z_{i\ (6\ hours)}$ | | |
|---|---|---|---|
| | 50 Hz | 500 Hz | 1,000 Hz |
| A*Escherichia coli* | 17 % | 15 % | 12 % |
| B*Escherichia coli* | 14 % | 11 % | 10 % |

As shown in the table, in this embodiment the change values obtained are slightly less sensitive than in the second embodiment, wherein the percentage of variation in capacitance is measured. However, determining the imaginary impedance component at a frequency has the advantage of substantially simplifying the detection process.

At 50 Hz the results reveal greater sensitivity, while the other values are slightly lower.

Despite the fact that three specific embodiments of the present invention have been described and represented, it is evident that a person skilled in the art may introduce variants and modifications or substitute details for other technically equivalent ones, without diverging from the sphere of protection defined by the attached claims.

For example, in all the described embodiments the impedance measurements have been carried out using a microelectronic chip 1 type sensor device. Nevertheless, any other equivalent microelectronic device such as, for example, a microelectronic device manufactured using thick-film microelectronic technology could be useful for performing the measurements of the present invention.

Likewise, in the described embodiments, adhesion of the bacteria onto the surface of the work electrode 2 has been carried out through the formation of a biofilm 4. Nevertheless, adhesion of the bacteria can be carried out using any other type of technique such as, for example, carrying out the functionalisation of the work electrode 3 surface through the direct adsorption of a compound, for example avidin, which facilitates adhesion of the previously biotinylated bacteria by means of avidin-biotin bonds.

The described embodiments relate to the detection of bacteriophages infecting *Pseudomonas putida* and somatic coliphages infecting *Escherichia coli.* Nevertheless, as mentioned earlier, the present invention can be applied to the detection and/or quantification of any type of bacterophage, provided that there is a bacterial host strain susceptible to being infected by said bacteriophage.

**Claims**

1. A process for detecting and/or quantifying bacteriophages capable of infecting a predetermined bacterial host strain, **characterised** in that it comprises the stages of:

   a) adhering bacteria from at least one host strain onto the surface of a work electrode (2) of a device comprising means for measuring electrical impedance;
   b) exposing the electrode (2) and adhered bacteria to a solution of the material being analysed which is susceptible to containing bacteriophages;
   c) incubating, together with the electrode (2), the solution of stage b) under predetermined conditions so that, if said bacteria are infected by bacteriophages, lysis of said bacteria adhered onto the electrode takes place;
   d) measuring the electrical impedance change of said solution, while carrying out the incubation of stage c),
   e) e-i) determining, in the equivalent electrical circuit, the change in capacitance value of the electrode-bacteria interface, based on said change in impedance value; or
   e-ii) determining the value of the change in magnitude of the imaginary impedance component at a predetermined frequency in accordance with said host strain, based on said change in impedance value; and
   f) determining the presence or concentration of bacteriophages in the solution, based on said change in capacitance value or change in magnitude of the imaginary impedance component, by means of the corresponding calibration curve that correlates said change in capacitance value or change in magnitude value of the imaginary impedance component, with the bacteriophage concentration of the solution.

2. A process according to claim 1, wherein the bacterial adhesion of stage a) comprises the formation of a bacterial biofilm (4) of at least one host strain on the surface said electrode (2).

3. A process according to claim 2, wherein:

   - stage e-i) comprises determining the change in capacitance (Cb) value of the biofilm adhered onto the electrode (2), said capacitance (Cb) being the parameter of the equivalent electrical circuit that said biofilm (4) models in the interface,

   and wherein, subsequent to stage e-i), in stage f) the presence or concentration of bacteriophages is determined based on the change in capacitance (Cb) of said biofilm (4), said change in capacitance being correlated with the deterioration of said biofilm (4) as a result of the lysis.

4. A process according to any of claims 2 to 3, wherein said biofilm (4) has a thickness of at least 25 microns or a bacterial concentration of said host strain of at least $10^4$ PFU/ml (Plaque-Forming Units).

5. A process according to any of claims 2 to 4, where in stage a) the formation of said biofilm (4) comprises the exposure of said electrode (2) to a culture medium containing bacteria from said host strain, and the simultaneous polarisation of said work electrode (2) for a predetermined period of time according to said host strain.

6. A process according to claim 1, wherein the bacterial adhesion of stage a) comprises the prior functionalisation of the surface of said electrode (2) through the adsorption of a compound destined for immobilising said bacteria on the surface of the electrode (2).

7. A process according to any of the preceding claims, wherein said work electrode (2) is integrated in the material substrate of a microelectronic device (1) which comprises at least another electrode (3) for performing electrical impedance measurements.

8. A system for detecting and/or quantifying bacteriophages capable of infecting a predetermined bacterial host strain, **characterised** in that it comprises:

   - a microelectronic sensor device (1) which includes at least two electrodes (2, 3) for performing electrical impedance measurements,

- bacteria from at least one host strain, adhered onto the surface of at least one of said electrodes (2), and
- processing and control means for determining either the change in capacitance of the electrode-bacteria interface in the equivalent circuit or the change in magnitude of the imaginary impedance component at a predetermined frequency according to said host strain,

said change in capacitance or change in magnitude of the imaginary impedance component being produced as a result of the lysis of the bacteria adhered onto the electrode (2), on being said bacteria infected by bacteriophages.

9. A system, according to claim 8, wherein said bacteria form part of a bacterial biofilm (4) adhered onto the surface of said electrode (2), and wherein said processing and control means either determine the change in capacitance (Cb) of the parameter of the equivalent electrical circuit modelled by said biofilm (4) in the interface or determine the change in magnitude of the imaginary impedance component, said change in capacitance (Cb) or said change in magnitude of the imaginary impedance component being correlated with the deterioration of said biofilm (4) as a result of the bacterial lysis.

10. A microelectronic device comprising at least one pair of electrodes (2,3) for measuring changes in electrical impedance, **characterised** in that it comprises a bacterial biofilm (4) adhered onto the surface of at least one of said electrodes (2), the bacteria of said biofilm (4) stemming from a host strain susceptible of being infected by bacteriophages having a predetermined specificity for said strain.

11. A device, according to claim 10, wherein said biofilm (4) has a thickness of at least 25 microns or a bacterial concentration of said host strain of at least $10^4$ PFU/ml (Plaque-Forming Units).

12. A device, according to any of claims 10 to 11, wherein said microelectronic device (1) is a sensor chip.

13. Use of a microelectronic sensor device (1) comprising at least one pair of electrodes (2,3) integrated in a material substrate, for detecting and/or quantifying, using electrochemical impedance spectroscopy, bacteriophages capable of infecting a predetermined bacterial host strain.

14. Use according to claim 13, wherein said device comprises a bacterial biofilm (4) adhered onto its surface, the bacteria of said biofilm (4) from at least one of said host strains, and said biofilm preferably having a thickness of at least 25 microns or a concentration of bacteria from said host strain of at least $10^4$ PFU/ml (Plaque-Forming Units).

15. A process according to claim 1, a system according to claim 8, a microelectronic device according to claim 10 or use of said microelectronic device according to claim 13, wherein said bacteriophages are somatic coliphages or bacteriophages capable of infecting strains of *Escherichia coli,* bacteriophages capable of infecting strains of lactic bacteria or bateriophages capable of infecting strains of *Pseudomonas putida.*

16. A process according to claim 1, a system according to claim 8, a microelectronic device according to claim 10 or use of said microelectronic device according to claim 13, wherein the material solution being analysed stems from a water sample.

FIG.1

FIG.2b

FIG.2a

FIG.3

FIG.4

# INTERNATIONAL SEARCH REPORT

| | International application No. |
|---|---|
| | PCT/ ES 2009/070066 |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

C12Q1/70, G01N33/00

Documentation searched other than minimum documentation to the extent that such documents are included in the fields searched

Electronic data base consulted during the international search (name of data base and, where practicable, search terms used)

INVENES,EPODOC

**C. DOCUMENTS CONSIDERED TO BE RELEVANT**

| Category* | Citation of document, with indication, where appropriate, of the relevant passages | Relevant to claim No. |
|---|---|---|
| Y | ES 2256028 T3 (PROFOS AG.) 16.07.2006, the whole Documento. | 1-18 |
| Y | WO 2007104058 A2 (WEISS, GREGORY A. et al.) 13.09.2007, the whole Documento. | 1-18 |
| A | ES 2137373 T3 (SANDERS, MICHAEL FREDERICK) 16.12.1999, the whole Documento. | 1-18 |
| A | WO 9317129 A (Judkins, Paul, W.) 02.09.1993, the whole Documento. | 1-18 |
| A | ES 2235329 T3 (ISTITUTO DI RICERCHE DI BIOLOGIA MOLECOLARE P. ANGELETTI ) 08.03.2000, the whole Documento. | 1-18 |
| A | EP 0149383 B1 (BOUTON NEUVY, LILIANE et al) 27.01.1988, the whole Documento. | 1-18 |

☐ Further documents are listed in the continuation of Box C.   ☒ See patent family annex.

| | |
|---|---|
| * Special categories of cited documents:<br>"A" document defining the general state of the art which is not considered to be of particular relevance.<br>"E" earlier document but published on or after the international filing date<br>"L" document which may throw doubts on priority claim(s) or which is cited to establish the publication date of another citation or other special reason (as specified)<br>"O" document referring to an oral disclosure use, exhibition, or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other documents , such combination being obvious to a person skilled in the art<br>"&" document member of the same patent family |
| Date of the actual completion of the international search<br><br>21 July 2009      (21.07.2009) | Date of mailing of the international search report<br><br>**(22/07/2009)** |
| Name and mailing address of the ISA/<br>O.E.P.M.<br><br>Paseo de la Castellana, 75 28071 Madrid, España.<br>Facsimile No.   34 91 3495304 | Authorized officer<br><br>G. Foncillas Garrido<br><br>Telephone No. +34 91 349 32 82 |

Form PCT/ISA/210 (second sheet) (July 2008)

## INTERNATIONAL SEARCH REPORT

Information on patent family members

| International application No. |
| --- |
| PCT/ ES 2009/070066 |

| Patent document cited in the search report | Publication date | Patent family member(s) | Publication date |
| --- | --- | --- | --- |
| EP 0714450 AB | 05.06.1996 | WO 9505483 A | 23.02.1995 |
| | | CA 2169168 AC | 23.02.1995 |
| | | AU 7235894 A | 14.03.1995 |
| | | EP 19940921764 | 29.07.1994 |
| | | GB 2295893 AB | 12.06.1996 |
| | | NZ 268957 A | 24.03.1997 |
| | | JP 9503653 T | 15.04.1997 |
| | | JP 3681389 B | 10.08.2005 |
| | | AU 680279 B | 24.07.1997 |
| | | US 5914240 A | 22.06.1999 |
| | | AT 185380 T | 15.10.1999 |
| | | ES 2137373 T | 16.12.1999 |
| | | DK 714450 T | 27.12.1999 |
| | | DE 69421085 T | 27.01.2000 |
| | | GR 3031746 T | 29.02.2000 |

Form PCT/ISA/210 (patent family annex) (July 2008)

| **INTERNATIONAL SEARCH REPORT** | International application No. |
| --- | --- |
| | PCT/ ES 2009/070066 |

CLASSIFICATION OF SUBJECT MATTER

*C12Q 1/70* (2006.01)
*G01N 33/00* (2006.01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0149383 A
- EP 0714450 A

- ES 200800109